# EUROPEAN PATENT APPLICATION

(11) **EP 3 597 775 A1**
(43) Date of publication of application: **22.01.2020**
(21) Application number: 18184111.5
(22) Date of filing: 18.07.2018
(51) Int. Cl.: C12Q 1/6823, C12Q 1/6825, C12Q 1/6834, C12N 15/115

(54) **METHOD FOR THE DETECTION AND QUANTIFICATION OF SMALL MOLECULES AND FLUIDIC PLATFORM FOR PERFORMING THE SAME**

(71) Applicant: Hochschule Furtwangen, 78120 Furtwangen im Schwarzwald (DE)
(72) Inventor: Deigner, Hans-Peter, 68623 Lampertheim (DE); Mahmoud, Mostafa Mohamed Safwat Ahmed, 78054 Villingen-Schwenningen (DE)
(74) Representative: Müller-Boré & Partner Patentanwälte PartG mbB

(57) **Abstract**

The present invention relates to methods for the detection and/or quantification of an analyte of interest in a fluid sample, comprising the use of analyte-specific RNA or DNA aptamers and displaceable oligonucleotides that bind to the binding region of said aptamers. The present invention further relates to microfluidic devices that can be used in said methods.

## Description

The present invention relates to methods for the detection and/or quantification of an analyte of interest in a fluid sample, comprising the use of analyte-specific RNA or DNA aptamers and displaceable oligonucleotides that bind to the binding region of said aptamers. The present invention further relates to microfluidic devices that can be used in said methods.

The detection and quantification of small molecule analytes in fluid samples remains a considerably challenging task that requires bulky and complex instruments as well as well-trained personnel, e.g. in the case of mass spectrometry. Their detection is hampered by the intrinsic properties of small molecule analytes, such as their small size and the availability of only one epitope for binding. Thus, most immunoassays still do not provide fast results and lack the sensitivity required for diagnostics. Moreover, the tedious steps included, e.g. washing, blocking and signal development, hinders the transfer of these assays to Point-of-Care (POC) devices.

Oligonucleotide aptamers stand out as a promising alternative to antibodies and similar specific proteinaceous binding molecules due to their stability, fast binding kinetics and reversibility of the binding. Aptamers are single stranded DNA or RNA sequences selected *in vitro* for binding to a target analyte, e.g. through a process known as SELEX (Systematic Evolution of Ligands by EXponential Enrichment). The fast binding kinetics and the easy functionalization of aptamers enables the development of non-conventional POC devices.

Magnetic beads are micro- or nano-sized particles containing iron or iron oxide. Magnetic mono-sized particles provide a large and uniform surface area for attaching functional molecules, such as antibodies, enzymes, DNA or aptamers. This can be achieved through various covalent and non-covalent strategies. The uniqueness of magnetic beads is the exceptionally fast separation of the same in fluid media using external magnetic forces. This allows for a hybrid assay which is fast due to the easy and efficient mixing and allows including washing steps when needed. These properties made magnetic beads suitable for use in various applications, such as cell preparation, immunoassays, bioseparation and targeted drug delivery.

Lateral flow assays are a class of paper or fiber based POC assays. Lateral flow immunoassays (LFIA) are simple, fast and can provide reliable results within a few minutes. Thus, lateral flow assays (LFA) or similar fluidic assays provide a rapid and cost-effective means to detect targets *in situ.* However, producing these type of test strips requires immobilization of antibodies or antigens on the surface of the test strips which can affect the conformation and stability of the reagents. Further, the commonly used sandwich or competitive formats are unsuitable for the detection of small molecules. This is due to the small size of the target molecules, hindering the efficient labeling for a competitive assay and the unavailability of two different binding epitopes required for sandwich assays.

Recently, a LFA system for small molecules based on adsorption and desorption on gold nanoparticles has been described. This assay made use of the fact that single stranded DNA adsorbs onto the surface of gold nanoparticles and when the target analyte is introduced, this ssDNA binds to it and desorbs from the surface. However, this assay, as well as other assay systems known in the art, is based on complex detection techniques and methodology.

Thus, there is a need to provide fast, easy, accurate and sensitive assay systems for the detection and quantification of small molecule analytes. These systems should not require the immobilization of proteins or any of the assay components, allow for easy detection by both visual and instrumental means, allow for regeneration of the assay components, and provide means for the detection and quantification of multiple different target analytes.

Therefore, the technical problem underlying the present invention is the provision of respective methods and devices for the detection and quantification of small molecule analytes.

The solution to the above technical problem is achieved by the embodiments characterized in the claims.

In particular, in a first aspect, the present invention relates to a method for the detection and/or quantification of an analyte of interest in a fluid sample, comprising the steps of:
(a) providing a fluid sample in a reaction vessel, wherein said fluid sample is to be analyzed for the presence and/or quantity of the analyte of interest;
(b) adding to said sample in said reaction vessel, either subsequently in any order, or concurrently,
   (i) an RNA or DNA aptamer, and
   (ii) an oligonucleotide,
   wherein
   said RNA or DNA aptamer is coupled to a magnetic bead and said oligonucleotide is coupled to a detectable marker; or said RNA or DNA aptamer is coupled to a detectable marker and said oligonucleotide is coupled to a magnetic bead,
   said RNA or DNA aptamer comprises a binding region that is capable of binding the analyte of interest with a first affinity, and
   said oligonucleotide comprises a nucleotide sequence that is complementary to a nucleotide sequence of said RNA or DNA aptamer that forms at least a part of the aptamer's binding region, wherein said nucleotide sequences bind to each other via Watson-Crick pairing with a second affinity, wherein said second affinity is lower than said first affinity;
(c) subjecting the reaction vessel to a magnetic field, so that said magnetic bead-coupled RNA or DNA aptamers or magnetic bead-coupled oligonucleotides are sequestered to an inner surface of the reaction vessel;
(d) detecting the presence or absence and/or determining the amount of the detectable marker in the sample supernatant; and
(e) determining the presence or absence and/or the amount of the analyte of interest in the sample, wherein
   the presence of detectable marker in the sample supernatant indicates that the RNA or DNA aptamer has bound the analyte of interest, so that the oligonucleotide has been displaced by the analyte of interest from the RNA or DNA aptamer or has been prevented from binding to the same, the magnetic bead-coupled RNA or DNA aptamer or the magnetic bead-coupled oligonucleotide has been sequestered to an inner surface of the reaction vessel, and the detectable maker-coupled oligonucleotide or the detectable maker-coupled RNA or DNA aptamer remains in the sample supernatant, thus indicating the presence of the analyte of interest in the sample, and
   the amount of detectable marker in the sample supernatant positively correlates with the amount of the analyte of interest in the sample.

As used herein, the term "detection" with regard to the analyte of interest relates to the mere determination of whether said analyte of interest is present or absent in the sample, whereas the term "quantification" with regard to said analyte relates to the determination of the actual amount of analyte in the sample.

The analyte of interest to be detected and/or quantified with the methods of the present invention is not particularly limited in any way, provided that it is an analyte for which an RNA or DNA aptamer binding specifically to it can be generated. Considering the fact that such aptamers can be generated for a wide range of targets, including small molecules, proteins, nucleic acids, and even cells, tissues and organisms, this does not constitute a significant restriction. In preferred embodiments, the analyte of interest is a small molecule, e.g. a low molecular weight organic compound, e.g. having a molecular weight of below about 900 Da. In further embodiments, the target analyte is a small molecule analyte selected from the group consisting of drugs, metabolites, toxins, environmental pollutants, and food contaminants, including target analytes of clinical and non-clinical relevance. In this context, the terms "analyte of interest", "target analyte" or simply "target" are used synonymously herein.

The fluid sample to be used in the present invention is not particularly limited. Suitable samples can be diluted or undiluted samples that are natively fluid, e.g. blood, blood serum, blood plasma, urine, cerebrospinal fluid, saliva, semen, environmental samples such as water samples, and fluid comestibles. Further, suitable samples can also derive from non-fluid samples that have been dissolved in a suitable solvent to bring them into a fluid state. Respective non-fluid sample materials include e.g. fecal matter, environmental samples such as soil samples, and solid comestibles. Suitable diluents and solvents are not particularly limited and are known in the art, wherein aqueous diluents and solvents, in particular water, are preferred.

In step (a) of the methods of the present invention, a fluid sample that is to be analyzed for the presence and/or quantity of the analyte of interest is provided in a reaction vessel. Suitable reaction vessels are not particularly limited, and include e.g. standard micro reaction tubes ("Eppendorf" tubes), microtiter plates, and the like.

In a specific embodiment, the reaction vessel is a microfluidic device, preferably a microfluidic device comprising:
(i) a fluid inlet and a fluid outlet,
(ii) a sequestering zone between the inlet and the outlet that is to be subjected to the magnetic field in step (c),
(iii) a detection zone between the sequestering zone and the outlet, comprising an absorbing pad, wherein step (d) is performed in said detection zone, and
(iv) microfluidic channels placing the inlet and the sequestering zone, the sequestering zone and the detection zone, and the detection zone and the outlet in fluid communication.

In embodiments using a respective microfluidic device, fluid flow from the fluid inlet towards the fluid outlet can be either controlled actively, e.g. by using respective pumps known in the art, or passively using capillary forces.

In preferred embodiments, the microfluidic device used in the methods of the present invention is the microfluidic device according to the second aspect of the present invention as defined hereinafter.

In step (b) of the methods of the present invention, an RNA or DNA aptamer and an oligonucleotide are added to the sample in the reaction vessel either subsequently, in any order, or concurrently, *i.e.,* either the RNA or DNA aptamer is added first, and then the oligonucleotide, the oligonucleotide is added first, and then the RNA or DNA aptamer, or both the RNA or DNA aptamer and the oligonucleotide are added concurrently. In specific embodiments, the RNA or DNA aptamer and the oligonucleotide are combined with each other, optionally incubated with each other for a brief time, and then concurrently added to the sample in the reaction vessel.

According to the present invention, one of the RNA or DNA aptamer and the oligonucleotide is coupled to a magnetic bead, and the other is coupled to the detectable marker, *i.e.,* said RNA or DNA aptamer is coupled to a magnetic bead and said oligonucleotide is coupled to a detectable marker; or said RNA or DNA aptamer is coupled to a detectable marker and said oligonucleotide is coupled to a magnetic bead. In preferred embodiments, the RNA or DNA aptamer is coupled to a magnetic bead and the oligonucleotide is coupled to a detectable marker. Methods of coupling aptamers and oligonucleotides to magnetic beads or detectable markers are not particularly limited and are known in the art. Coupling includes direct coupling, *i.e.,* forming a covalent or non-covalent (e.g. ionic, van der Waals or hydrogen) bond between the coupling partners, or indirect coupling via the use of binding systems known in the art, e.g. the biotin-streptavidin system. In specific embodiments, the RNA or DNA aptamers and oligonucleotides employed in the present invention are biotinylated, e.g. in the 3' position, and the magnetic beads and detectable markers are coated with streptavidin, thus facilitating binding of the former to the latter.

Further, said RNA or DNA aptamer comprises a binding region that is capable of binding the analyte of interest with a first affinity, and said oligonucleotide comprises a nucleotide sequence that is complementary to a nucleotide sequence of said RNA or DNA aptamer that forms at least a part of the aptamer's binding region, wherein said nucleotide sequences bind to each other via Watson-Crick pairing with a second affinity, wherein said second affinity is lower than said first affinity. In this context, it should be noted that binding affinities of RNA or DNA aptamers to their binding partner are generally much larger than the Watson-Crick binding affinities of short oligonucleotide stretches to each other, so that the latter prerequisite is generally easily fulfilled. Methods of generating RNA or DNA aptamers binding to a desired analyte of interest are not particularly limited and are known in the art. They include the SELEX method known in the art. Further, the generation of oligonucleotides that comprise a nucleotide sequence that is complementary to a nucleotide sequence of said RNA or DNA aptamer that forms at least a part of the aptamer's binding region is known in the art. In preferred embodiments, the oligonucleotide is a DNA oligonucleotide.

Detectable markers that can be used in the methods of the present invention are not particularly limited and are known in the art. They include e.g. fluorophores, colored particles such as e.g. dyed microbeads, quantum dots, and chromogenic enzymes that catalyze a color reaction, such as e.g. horseradish peroxidase. In preferred embodiments, the detectable marker is a marker advantageously allowing visual detection, e.g. by the naked eye, such as e.g. dyed microbeads.

The oligonucleotide's nucleotide sequence that is complementary to a nucleotide sequence of the RNA or DNA aptamer that forms at least a part of the aptamer's binding region has preferably a length of 5 to 15, more preferably 6 to 14, 7 to 13, 8 to 12, or 9 to 11 nucleotides, most preferably of about 10 nucleotides. In specific embodiments, the oligonucleotide consists entirely of this nucleotide sequence, *i.e.,* the oligonucleotide has a length of 5 to 15, more preferably 6 to 14, 7 to 13, 8 to 12, or 9 to 11 nucleotides, most preferably of about 10 nucleotides. In this context, said length can vary depending on the used aptamer and the affinity to the target. Further, said length can be easily optimized to achieve the condition that the above second affinity is lower than the above first affinity. This can include by varying the above length and/or including nucleotide mismatches Methods to determine the respective binding strengths are known in the art and include e.g. the use of common DNA thermodynamics analysis in order to determine the affinity of the complementary oligonucleotide to the aptamer and optimize it.

Further, the part of the DNA or RNA aptamer's binding region to which the oligonucleotide is complementary preferably has a length of 3 to 20, more preferably 3 to 17, 4 to 16, 5 to 15, 6 to 14, 7 to 13, 8 to 12, or 9 to 11 nucleotides, most preferably of about 10 nucleotides.

In step (c) of the methods of the present invention, the reaction vessel is subjected to a magnetic field, e.g. by placing the reaction vessel in direct contact with a magnet or by placing the reaction vessel in the vicinity of a magnet, so that the magnetic bead-coupled moiety, i.e. the magnetic bead-coupled RNA or DNA aptamers or the magnetic bead-coupled oligonucleotides are sequestered to an inner surface of the reaction vessel.

In this manner, in case the analyte of interest is not present in the sample, the entire complex of magnetic bead-coupled moiety and detectable marker-coupled moiety, bound to each other via Watson-Crick pairing of the oligonucleotide to the RNA or DNA aptamer, is sequestered to the inner surface of the reaction vessel, so that no detectable marker remains in the sample supernatant. However, in case the analyte of interest is present in the sample, the analyte displaces the oligonucleotide from the aptamer, or the analyte prevents the oligonucleotide from binding to the aptamer in the first place, due to the higher binding affinity of analyte to aptamer as compared to the affinity of Watson-Crick pairing of oligonucleotide to aptamer, so that the magnetic bead-coupled moiety, *i.e.,* either magnetic bead-coupled aptamer with bound analyte or magnetic bead-coupled oligonucleotide, is sequestered to the inner surface of the reaction vessel, whereas the detectable marker-coupled moiety, *i.e.,* the detectable marker-coupled oligonucleotide or the detectable maker-coupled aptamer with bound analyte, remain in the sample supernatant and can be detected therein. In this context, the term "sample supernatant" refers to the fluid material in the reaction vessel from which the magnetic bead-coupled moieties have been removed by sequestering the same to an inner surface of the reaction vessel.

In step (d) of the methods of the present invention, the presence or absence of the detectable marker in the sample supernatant is detected and/or the amount of the detectable marker in the sample supernatant is determined. Means for detecting a given detectable marker are not particularly limited and are known in the art. They include any suitable visual or instrumental means. In specific embodiments, the detectable marker is a dyed microbead, advantageously allowing visual detection of the marker in the sample supernatant, e.g. by the naked eye. In case the detectable marker is a chromogenic enzyme catalyzing a color reaction, step (d) of the methods of the present invention encompasses the addition of a suitable substrate, such as e.g. 3,3',5,5'-tetramethylbenzidin or o-phenylenediamine dihydrochloride in the case of horseradish peroxidase. Further, means for determining the amount of detectable marker in the sample supernatant for a given marker are not particularly limited and are known in the art. They include e.g. photometric (absorbance) measurements and the use of calibration curves using known amounts of analyte, the detection of light emission of fluorophores as detectable marker, as well as other suitable instrumental means known in the art.

In step (e) of the methods of the present invention, the presence or absence of the analyte of interest and/or the amount of the analyte of interest in the sample are determined, wherein the presence of detectable marker in the sample supernatant indicates that the RNA or DNA aptamer has bound the analyte of interest, so that the oligonucleotide has been displaced by the analyte of interest from the RNA or DNA aptamer or has been prevented from binding to the same, the magnetic bead-coupled RNA or DNA aptamer or the magnetic bead-coupled oligonucleotide has been sequestered to an inner surface of the reaction vessel, and the detectable maker-coupled oligonucleotide or the detectable maker-coupled RNA or DNA aptamer remains in the sample supernatant, thus indicating the presence of the analyte of interest in the sample. Further, the amount of detectable marker in the sample supernatant positively correlates with the amount of the analyte of interest in the sample.

So far herein, the methods of the present invention have been described for the detection of one analyte of interest, using one pair of RNA or DNA aptamer and oligonucleotide (oligonucleotide/aptamer pair). However, in specific embodiments, the methods of the present invention can be used for the concurrent detection and/or quantification of more than one analyte of interest in the fluid sample. In these embodiments, step (b) of the methods of the present invention comprises the addition of one oligonucleotide/aptamer pair for each analyte of interest, wherein the detectable markers of each oligonucleotide/aptamer pair can be distinguished from each other, step (d) of the methods of the present invention comprises the detection of the presence or absence and/or the determination of the amount of each detectable marker, and step (e) of the methods of the present invention comprises determining the presence or absence and/or the amount of each analyte of interest in the sample.

Means for the detection and/or quantification of more than one detectable markers in the sample supernatant are not particularly limited and are known in the art. They include e.g. photometric (absorbance) measurements at different wavelengths, the detection of light emission of different fluorophores as detectable marker as well as any other suitable instrumental means known in the art. Further, also visual detection of e.g. different dyed microbeads can be used in this respect, provided that these differ in a further characteristic allowing separation of different dyed microbeads from each other. This characteristic may be the size of the microbeads, allowing for embodiments including the separation of different sized microbeads, e.g. in the devices of the present invention. Respective separation methods are not particularly limited and are known in the art.

The methods of the present invention are preferably *in vitro* methods, *i.e*., they are not performed on the human or animal body.

In a second aspect, the present invention relates to a microfluidic device, comprising
(i) a fluid inlet and a fluid outlet,
(ii) a sequestering zone between the inlet and the outlet that is to be subjected to the magnetic field in step (c),
(iii) a detection zone between the sequestering zone and the outlet, comprising an absorbing pad, wherein step (d) is performed in said detection zone, and
(iv) microfluidic channels placing the inlet and the sequestering zone, the sequestering zone and the detection zone, and the detection zone and the outlet in fluid communication.

The sequestering zone and the detection zone of the microfluidic device of the present invention are preferably fluid reservoirs formed within the device. Further, materials for forming the absorbing pad in the detection zone are not particularly limited and are known in the art. They include e.g. cellulose.

Fluid flow from the fluid inlet towards the fluid outlet can be either controlled actively, e.g. by using respective pumps known in the art, or passively using capillary forces.

The term "about" as used herein refers to a modification of the specified amount of ± 10%, preferably ± 5, 3, 2, or 1%. By way of example, the term "about 900 Da" can refer to a molecular weight of 810 to 990 Da, and the term "about 10" can refer to a range of 9 to 11.

The term "comprise(s)/comprising" as used herein is expressly intended to encompass the terms "consist(s)/consisting essentially of" and "consist(s)/consisting of".

The present invention provides an assay system based on strand displacement and a combination of magnetic and dyed microbeads. This system mimics a lateral flow system, however, there is no need to immobilize the reagents on a test strip or cassette and the assay allows for a combination of different detectable markers for multiplexing. In contrast to most competitive assays known in the art, wherein the signal is inversely related to the concentration of the target, in the methods of the present inveniton, the signal intensity in the sample supernatant is positively correlated with the amount of target analyte.

The aim of the present invention was the development of a fast quantitive assay for small molecules such as drugs, metabolites, toxins and environmental pollutants. This assay is also able to work with minimum equipment or none equipment at all, and allows non-trained personnel and home users to use it through visual detection. Thus, it will have a great potential for various applications, such as e.g. the detection and quantification of drugs of abuse or intoxication, environmental or food contaminants and clinical applications in a simple POC format.

The principle of the assay of the present invention is making use of strand displacement, where an aptamer specific for a certain target analyte is used in combination with a complementary strand. This complementary strand competes with the target and thus can be used for quantification.

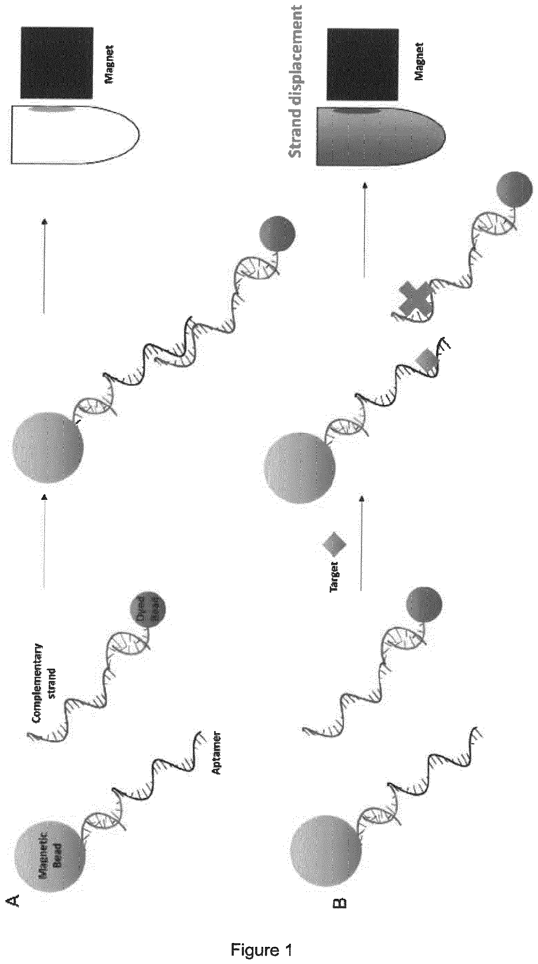

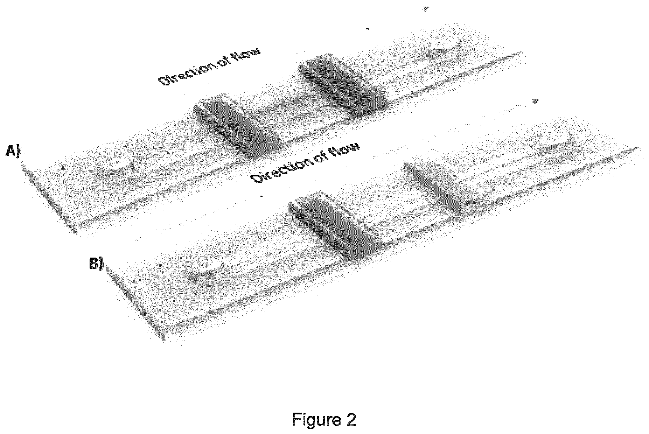

The assay principle is shown in detail in Figure 1. The detection molecule here is a blue micro bead that allows the quantification either visually or by measuring the absorbance. However, the assay is very flexible and can make use of other detection molecules such as quantum dots. This allows for easy multiplexing. The assay of the present invention can be used in connection with a microfluidic device (Figure 2) and combined with a POC detector for quantification. The parameters of such device and its design are adapted and optimized in such a way that no user interference is necessary.

In comparison to methods known in the art, the methods of the present invention do not require the immobilization of proteins or any of the assay components on e.g. a test strip. Moreover, the detection can be both visual and instrumental through measuring absorbance. The assay components can be regenerated, e.g. the sensor magnetic beads and the fluidic device after washing. The principle of the present invention allows for multiplexing through changing the label to e.g. quantum dots or similar or even using colored particles with different absorbance wavelength. The assay kinetics and flow speeds can be adapted through active fluidic control (pump) allowing faster results.

The figures show:
Figure 1:
   Schematic illustration of one embodiment of the assay of the present invention. (A) represents the reaction in absence of the target molecules. The complementarity between the aptamer and the complementary strand leads to a clear solution when the beads are magnetically separated. (B) Upon addition of the target analyte, the complementary strand is displaced from the aptamer or is prevented from binding to the aptamer in the first place, leading to a concentration-dependent increase in the blue color of the solution.
Figure 2:
   Schematic representation of the microfluidic device of the present invention. (A) represents the assay in presence of target analyte, the brown rectangle represents a permanent magnet to be placed below the device where the other rectangle is a cellulose or similar absorbing pad to be placed within the device. (B) represents the negative assay in absence of the target analyte. The flow could be either controlled actively (pumps) or passively using capillarity or similar means.
Figure 3:
   The figure shows a control experiment to exclude non-specific adsorption. The right Eppendorf tube shows a negative control where the beads were functionalized by non-complementary oligonucleotides whereas the left one had an ethanolamine aptamer and its corresponding complementary strand. As seen in the right Eppendorf tube, the beads did not attach to each other and the solution remained blue. On the other hand, the complementarity between the aptamer and the short strand lead to the attachment of the beads together and the solution is clear.
Figure 4:
   The assay of the present invention performed in Eppendorf tubes, wherein the right tube is the negative control (no target), the middle is 10 nM of ethanolamine (610 picograms/ml), and the left is 100 nM (6 ng/ml). The color intensity between the negative and positive assays could be clearly distinguished by the naked eye.
Figure 5:
   Calibration curve for the absorbance of blue beads measured at a wavelength of 600 nm. The absorbance is plotted against the concentration and the line represents the best fitting line (r² =0.99). The error bars represent the standard deviation, n=3.

The present invention will be further illustrated by the following example without being limited thereto.

### Example

### Material

Aptamers and oligonucleotides were purchased from Integrated DNA Technology (Coralville, IA).

| Name | Ethanolamine aptamer |
|---|---|
| Sequence | ATTCAATTTGAGGCGGGTGGGTGGGTTGAAT/3Bio/ (SEQ ID NO: 1) |
| 5' Modification | none |
| 3' Modification | Biotin |
| Supplier | Integrated DNA Technology (Coralville, IA) |
| Concentration | 100 µM |
| Molecular weight | 101 kDa |

| Name | Et 10_3 (complementary strand) |
|---|---|
| Sequence | CCACCCACCC/3Bio/ (SEQ ID NO: 2) |
| 5' Modification | none |
| 3' Modification | Biotin |
| Supplier | Integrated DNA Technology (Coralville, IA) |
| Concentration | 100 µM |
| Molecular weight | 33 kDa |

The microparticles were supplied by Bangs Labs Inc., Indiana, USA as follows:

| Name | Streptavidin Coated Magnetic Classical |
|---|---|
| Mean diameter | 0.36 µm |
| Binding capacity | 1.24 µM biotin/mg microspheres |
| Concentration | 10 mg/ml |

| Name | Streptavidin Coated Microspheres (Cabo Blue) |
|---|---|
| Mean diameter | 0.192 µm |
| Binding capacity | 2.55 µM biotin/mg microspheres |
| Concentration | 10 mg/ml |

All other chemicals used herein were purchased from standard suppliers and used without further purification.

The functionalization of the beads was done according to the manufacturer's instructions.

### Example:

First, streptavidin-coated beads were reacted with the corresponding biotinylated oligonucleotide, wherein the magnetic beads were functionalized with the ethanolamine aptamer and the blue beads with the complementary strand.

The aptamer was selected and optimized as known in the art. The respective aptamer is well characterized and is specific for ethanolamine which is a very small organic molecule (Mw = 61.08 g/mol) and represent one of the smallest aptamer targets so far which makes it a very suitable model to prove the feasibility of the concept underlying the present invention.

To prove the success of the reaction and to exclude any non-specific adsorption, a negative control with the same exact beads and handling procedures was made. However, the beads were functionalized with non-complementary sequences and therefore should lack the ability to bind each other.

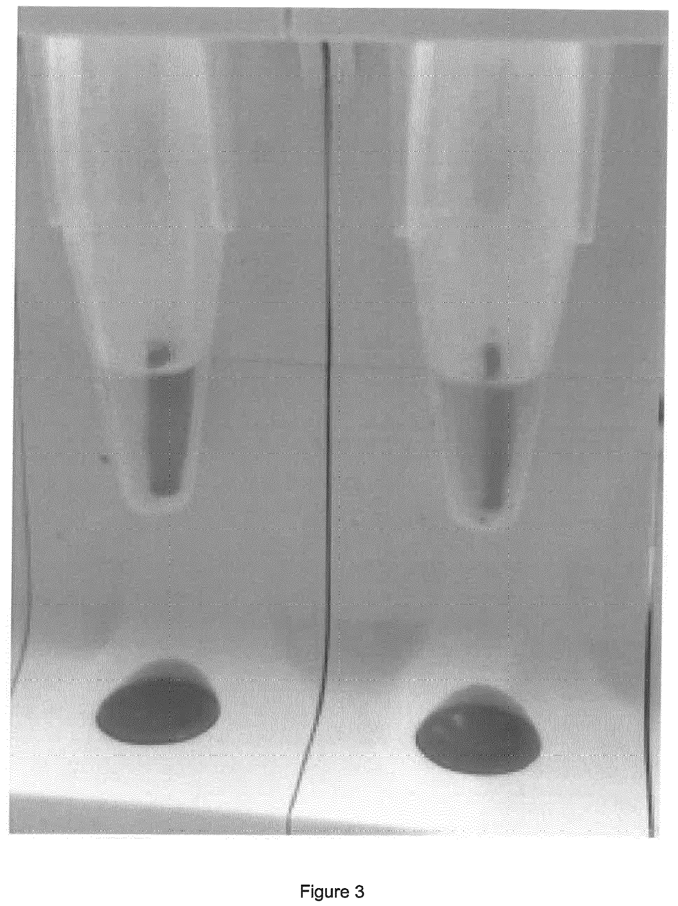

As shown in Figure 3, the beads were successfully functionalized and the non-specific adsorption was minimal. The results also revealed that the volume should be decreased in order to increase the color intensity.

Therefore, in order to develop the sensor, the functionalized magnetic beads and blue beads were allowed first to react together. Then, the supernatant was discarded to leave a magnetic beads-blue beads sensor. Then this was reacted with the target (50 µL) at 100 nM and 10 nM of ethanolamine.

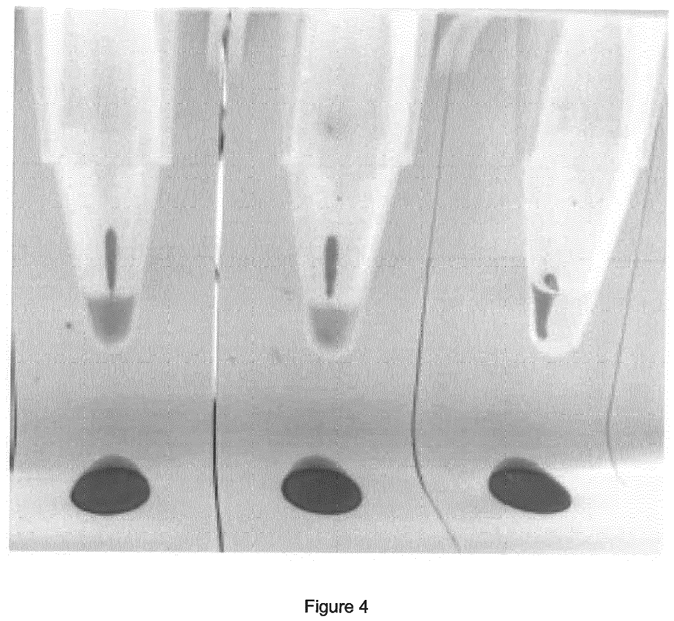

As shown in Figure 4, the 10 nM concentration lead to a visual color change in comparison to the control. With this setup, 10 nM of ethanolamine which corresponds to a concentration of 610 picograms/ml could be detected.

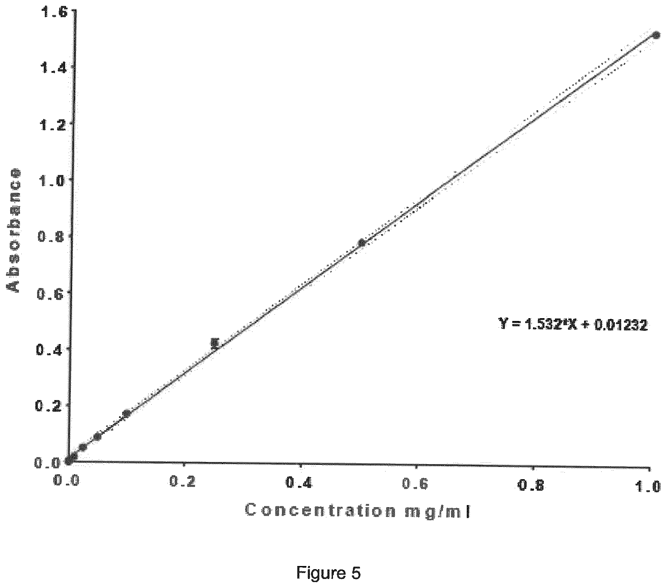

Further, an absorption calibration curve for the blue beads was established (Figure 5). This means that the reaction could also be quantified through the use of a conventional absorbance detector.

## Claims

1. A method for the detection and/or quantification of an analyte of interest in a fluid sample, comprising the steps of:
(a) providing a fluid sample in a reaction vessel, wherein said fluid sample is to be analyzed for the presence and/or quantity of the analyte of interest;
(b) adding to said sample in said reaction vessel, either subsequently in any order, or concurrently,
(i) an RNA or DNA aptamer, and
(ii) an oligonucleotide,
wherein
said RNA or DNA aptamer is coupled to a magnetic bead and said oligonucleotide is coupled to a detectable marker; or said RNA or DNA aptamer is coupled to a detectable marker and said oligonucleotide is coupled to a magnetic bead,
said RNA or DNA aptamer comprises a binding region that is capable of binding the analyte of interest with a first affinity, and
said oligonucleotide comprises a nucleotide sequence that is complementary to a nucleotide sequence of said RNA or DNA aptamer that forms at least a part of the aptamer's binding region, wherein said nucleotide sequences bind to each other via Watson-Crick pairing with a second affinity,
wherein said second affinity is lower than said first affinity;
(c) subjecting the reaction vessel to a magnetic field, so that said magnetic bead-coupled RNA or DNA aptamers or magnetic bead-coupled oligonucleotides are sequestered to an inner surface of the reaction vessel;
(d) detecting the presence or absence and/or determining the amount of the detectable marker in the sample supernatant; and
(e) determining the presence or absence and/or the amount of the analyte of interest in the sample, wherein
the presence of detectable marker in the sample supernatant indicates that the RNA or DNA aptamer has bound the analyte of interest, so that the oligonucleotide has been displaced by the analyte of interest from the RNA or DNA aptamer or has been prevented from binding to the same, the magnetic bead-coupled RNA or DNA aptamer or the magnetic bead-coupled oligonucleotide has been sequestered to an inner surface of the reaction vessel, and the detectable maker-coupled oligonucleotide or the detectable maker-coupled RNA or DNA aptamer remains in the sample supernatant, thus indicating the presence of the analyte of interest in the sample, and
the amount of detectable marker in the sample supernatant positively correlates with the amount of the analyte of interest in the sample.

2. The method of claim 1, wherein the analyte of interest is a small molecule analyte.

3. The method of claim 2, wherein the small molecule analyte is selected from the group consisting of drugs, metabolites, toxins, environmental pollutants, and food contaminants.

4. The method of any one of claims 1 to 3, wherein the fluid sample is selected from the group consisting of diluted or undiluted samples, selected from the group consisting of blood, blood serum, blood plasma, urine, cerebrospinal fluid, saliva, semen, environmental samples, and fluid comestibles, and dissolved non-fluid sample materials, selected from the group consisting of fecal matter, environmental samples, and solid comestibles.

5. The method of any one of claims 1 to 4, wherein the oligonucleotide is a DNA oligonucleotide.

6. The method of any one of claims 1 to 5, wherein the detectable marker is selected from the group consisting of fluorophores, colored particles, dyed microbeads, quantum dots, chromogenic enzymes, and quantum dots.

7. The method of any one of claims 1 to 6, wherein the oligonucleotide's nucleotide sequence that is complementary to a nucleotide sequence of said RNA or DNA aptamer that forms at least a part of the aptamer's binding region has a length of 5 to 15 nucleotides.

8. The method of any one of claims 1 to 7, wherein the part of the DNA or RNA aptamer's binding region to which the oligonucleotide is complementary has a length of 3 to 20 nucleotides.

9. The method of any one of claims 1 to 8, wherein the reaction vessel is a microfluidic device, said device comprising:
(i) a fluid inlet and a fluid outlet,
(ii) a sequestering zone between the inlet and the outlet that is to be subjected to the magnetic field in step (c),
(iii) a detection zone between the sequestering zone and the outlet, comprising an absorbing pad, wherein step (d) is performed in said detection zone, and
(iv) microfluidic channels placing the inlet and the sequestering zone, the sequestering zone and the detection zone, and the detection zone and the outlet in fluid communication.

10. The method of any one of claims 1 to 9, wherein more than one analyte of interest is detected and/or quantified in the fluid sample, wherein
step (b) comprises the addition of one oligonucleotide/aptamer pair for each analyte of interest, wherein the detectable markers of each oligonucleotide/aptamer pair can be distinguished from each other,
step (d) comprises the detection of the presence or absence and/or the determination of the amount of each detectable marker, and
step (e) comprises determining the presence or absence and/or the amount of each analyte of interest in the sample.

11. The method of any one of claims 1 to 10, wherein said RNA or DNA aptamer is coupled to a magnetic bead and said oligonucleotide is coupled to a detectable marker.

12. A microfluidic device, comprising
(i) a fluid inlet and a fluid outlet,
(ii) a sequestering zone between the inlet and the outlet that is to be subjected to the magnetic field in step (c),
(iii) a detection zone between the sequestering zone and the outlet, comprising an absorbing pad, wherein step (d) is performed in said detection zone, and
(iv) microfluidic channels placing the inlet and the sequestering zone, the sequestering zone and the detection zone, and the detection zone and the outlet in fluid communication.


SEQUENCE LISTING

<110>  Hochschule Furtwangen

<120>  Method for the Detection and Quantification of Small Molecules
       and Fluidic Platform for Performing the Same

<130>  F 2335EU

<160>  2

<170>  PatentIn version 3.5

<210>  1
<211>  31
<212>  DNA
<213>  Artificial sequence

<220>
<223>  DNA aptamer

<220>
<221>  misc_feature
<222>  (31)..(31)
<223>  3' biotinylated

<400>  1
attcaatttg aggcgggtgg gtgggttgaa t                                    31

<210>  2
<211>  10
<212>  DNA
<213>  Artificial sequence

<220>
<223>  complementary sequence

<220>
<221>  misc_feature
<222>  (10)..(10)
<223>  3' biotinylated

<400>  2
ccacccaccc                                                            10

Seite 1

SEQUENCE LISTING

<110> Hochschule Furtwangen

<120> Method for the Detection and Quantification of Small Molecules
      and Fluidic Platform for Performing the Same

<130> F 2335EU

<160> 2

<170> PatentIn version 3.5

<210> 1
<211> 31
<212> DNA
<213> Artificial sequence

<220>
<223> DNA aptamer


<220>
<221> misc_feature
<222> (31)..(31)
<223> 3' biotinylated

<400> 1
attcaatttg aggcgggtgg gtgggttgaa t                                    31


<210> 2
<211> 10
<212> DNA
<213> Artificial sequence

<220>
<223> complementary sequence


<220>
<221> misc_feature
<222> (10)..(10)
<223> 3' biotinylated

<400> 2
ccacccaccc                                                           10